(19) Europäisches Patentamt
European Patent Office
Office européen des brevets

(11) Publication number: **0 168 876 B1**

(12) **EUROPEAN PATENT SPECIFICATION**

(45) Date of publication of patent specification: **16.08.89**

(51) Int. Cl.$^4$: **C 07 C 139/00,** C 07 C 143/44

(21) Application number: **85201049.5**

(22) Date of filing: **01.07.85**

# (54) Process for the preparation of carboxylic acid esters of hydroxy sulfonates.

(30) Priority: **17.07.84 GB 8418135**

(43) Date of publication of application: **22.01.86 Bulletin 86/04**

(45) Publication of the grant of the patent: **16.08.89 Bulletin 89/33**

(84) Designated Contracting States: **BE CH DE FR GB IT LI NL SE**

(56) References cited: **EP-A-0 106 379**

(73) Proprietor: **SHELL INTERNATIONALE RESEARCH MAATSCHAPPIJ B.V.**
**Carel van Bylandtlaan 30**
**NL-2596 HR Den Haag (NL)**

(72) Inventor: **Drent, Eit**
**Badhuisweg 3**
**NL-1031 CM Amsterdam (NL)**
Inventor: **van Broekhoven, Johannes Adrianus Maria**
**Badhuisweg 3**
**NL-1031 CM Amsterdam (NL)**

(74) Representative: **Aalbers, Onno et al**
**P.O. Box 302**
**NL-2501 CH The Hague (NL)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The invention relates to the preparation of carboxylic acid esters of hydroxy sulfonates by a carbonylation process.

Carboxylic acid esters of hydroxy sulfonates are compounds which may be used as surface active agents or as bleach activators in detergent compositions.

It is known to prepare these compounds by reacting hydroxy sulfonates with carboxylic acids employing conventional esterification methods.

European Patent Application No. 83201237.1 (Publication No. 0106379) is concerned with a process for the carbonylation of olefins in the presence of water, an alcohol or a carboxylic acid using a catalyst comprising a palladium compound, at least 5 mol of a triaryl phosphine per gram atom of palladium and an acid having a pKa < 2 (at 18°C in aqueous solution), except hydrohalogenic and carboxylic acids, as promotor. The reaction products are carboxylic acids, esters or carboxylic acid anhydrides. Thus, methylpropionate may be prepared by carbonylation of ethylene in the presence of methanol.

It has now been found that carboxylic acid esters of hydroxy sulfonates can be prepared by carbonylation of olefins in the presence of hydroxy sulfonates and an aromatic reaction medium using the catalytic system of European Patent Application No. 83201237.1 (Publication No. 0106379). In these aromatic media the carboxylic acid esters of hydroxy sulfates can be obtained in the form of a slurry or suspension, thereby allowing easy recovery of the homogeneous catalyst system.

The invention therefore relates to a process for the preparation of carboxylic acid esters of hydroxy sulfonates in which an olefinically unsaturated compound is carbonylated with carbon monoxide in the presence of an aromatic hydrocarbon reaction medium, a hydroxy sulfonate of the general formula (I) $HO—R—SO_3M$, wherein R represents a divalent optionaly substituted hydrocarbon group and M represents a metal of Group I and II of the Periodic Table of the Elements, palladium catalyst, at least 5 mol of a phosphine of the general formula (II) $PR^1R^2R^3$ wherein $R^1$, $R^2$ and $R^3$ each represent an optionally substituted aryl group, per gram atom of palladium and as promotor an acid having a pKa < 2, except hydrohalogenic and carboxylic acids, and in that the obtained solid carboxylic acid ester of hydroxy sulfonate is separated from the reaction mixture.

The olefinically unsaturated compound which can be carbonylated according to the invention may be an unsubstituted or a substituted alkene or cycloalkene preferably having 2—30, and in particular 2—20, carbon atoms and preferably 1—3 double bonds. The alkene or cycloalkene may be substituted, for instance, with one or more halogen atoms or cyano, ester, alkoxy, hydroxy, carboxy or aryl groups. If the substituents are not inert under the reaction conditions, the carbonylation reaction may be accompanied with other reactions. For instance, the carbonylation of a hydroxy group containing alkene is accompanied with esterification of the hydroxy group. Examples of suitable olefinic coompounds are ethene, propene, butene-1, butene-2, isobutene, the isomeric pentenes, hexenes, octenes and dodecenes, such as 2-methylbutene-1, 3-methylbutene-1, 3,3-dimethylbutene-1, 3-methylpentene-1, 3,3-dimethylpentene-1, 2,4,4-trimethylpentene-1, 2,5-dimethylhexene-1 and 2-ethylhexene-1, cyclooctadiene-(1,5), cyclododecene, cyclododecatriene-(1,5,9), allyl alcohol, methyl, acrylate, ethyl acrylate, methyl methacrylate, acrylnitrile, acrylamide, N,N-dimethyl acrylamide, vinyl chloride, allyl chloride, acrolein, oleic acid, methyl allyl ether and styrene.

The hydroxy sulfonates of the general formula (I) $HO—R—SO_3M$ to be used in the process of the invention are those in which R represents a divalent hydrocarbon group containing preferably not more than 20 carbon atoms. The hydrocarbon group may be aliphatic, cycloaliphatic or aromatic and may be substituted with one or more substituents, such as mentioned hereinbefore in connection with the olefinically unsaturated compounds to be used as starting material. The symbol M of the general formula (I) may represent a metal of Group I and II of the Periodic Table of the Elements such as Na, K, Cs, Ca, Mg, Cu or Zn. Preferably M represents the alkali metals Na or K.

Examples of suitable hydroxy sulfonates are sodium or potassium salts of 2-hydroxyethanesulfonic acid, 3-hydroxypropanesulfonic acid, 3-hydroxybutanesulfonic acid, 4-hydroxybenzenesulfonic acid, 2-hydroxybenzenesulfonic acid, 4-hydroxy-3-methylbenzenesulfonic acid, 4-hydroxy-2,6-dimethylbenzene-sulfonic acid, 5-hydroxy-3-methyl-2-propylbenzenesulfonic acid, 4-hydroxy-3-methoxybenzenesulfonic acid, 5-hydroxy-4-methoxy-2-methylbenzenesulfonic acid, 2-hydroxy-6-nonylbenzenesulfonic acid and 2,4-dihydroxybenzenesulfonic acid. The use of hydroxy sulfonates of the general formula I, wherein R represents a phenylene group is preferred.

Palladium catalysts suitable to be used in the process according to the invention are the salts of palladium with, for instance, nitric acid, sulphuric acid or alkane carboxylic acids having not more than 12 carbon atoms. Salts of hydrohalogenic acids may, in principle, be used as well, but are not preferred because they tend to counteract the effect of the promoter as defined hereinafter. A catalyst used by preference is palladium acetate. Moreover, palladium complexes may be used, for instance palladium acetylacetonate, tetrakistriphenylphosphinepalladium, bis-tri-o-tolylphosphinepalladium acetate or bis-triphenylphosphinepalladium sulphate.

The quantity of palladium catalyst may vary within wide ranges. Preference is given to the use of quantities in the range between $10^{-5}$ and $10^{-1}$ gram atom palladium per mol of olefinically unsaturated compound.

The substituted or unsubstituted aryl groups $R^1$, $R^2$ and $R^3$ of the phosphine $PR^1R^2R^3$ preferably contain not more than 18, in particular 6—14, carbon atoms. Examples of suitable $R^1$, $R^2$ and $R^3$ groups are the naphthyl group and in particular the phenyl group. Suitable substituents are halogen atoms and alkyl, aryl, alkoxy, carboxy, carbaloxy, acyl, trihalogenmethyl, cyano, dialkylamino, sulphonylalkyl and alkanoyloxy groups.

Examples of suitable phosphines are tri-p-tolylphosphine, tri-p-methoxyphenylphosphine, o-diphenylphosphinobenzoic acid and in particular triphenylphosphine. The phosphine is used in a quantity of at least 5 mol, preferably at least 10 mol per gram atom of palladium. If the palladium catalyst already contains phosphine, this should be taken into account when calculating the amount of phosphine to be used.

The acids used as promoters in the process according to the invention preferably have a non-coordinating anion, by which is meant that little or no co-valent interaction takes palce between the palladium and the anion. Typical examples of such anions are $PF_6^-$, $SbF_6^-$, $BF_4^-$ and $ClO_4^-$.

Acids preferably used are, for instance, sulphonic acids and those acids that can be formed, possibly in situ, by interaction of a Lewis acid such as, for example, $BF_3$, $AsF_5$, $SbF_5$, $PF_5$, $TaF_5$ or $NbF_5$ with a Broensted acid such as, for example, a hydrohalogenic acid, in particular HF, fluorosulfonic, phosphoric acid or sulfuric acid. Specific examples of the last-named type of acids are fluorosilicic acid, $HBF_4$, $HPF_6$ and $HSbF_6$. Typical sulfonic acids that can be used are fluorosulfonic acid, chlorosulfonic acid and the sulfonic acids specified hereinafter.

A preferred group of acids has the general formula

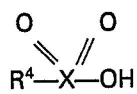

III

wherein X represents sulfur or chlorine and, if X is chlorine, $R^4$ represents oxygen and if X is sulfur, $R^4$ represents an OH group or an optionally substituted hydrocarbon group.

When the afore-mentioned acids are used in the process according to the invention, the anions of the acids can be regarded as non-coordinating.

In the acids having the general formula III, the optionally substituted hydrocarbon group, represented by $R^4$, is preferably an alkyl, aryl, aralkyl or alkaryl group with 1—30, in particular 1—14, carbon atoms. The hydrocarbon group may be substituted with for instance halogen atoms, in particular fluorine atoms, hydroxy groups or acyl groups. Examples of suitable acids of the general formula III are perchloric acid, sulfuric acid, 2-hydroxypropane-2-sulfonic acid, p-toluene-sulfonic acid and trifluoromethane sulfonic acid.

Further suitable sulfonic acid promotors are the hydroxy sulfonic acids and the carboxylic esters thereof corresponding respectively with the hydroxy sulfonates used as starting material and the carboxylic esters obtained as product in the process such as for instance's 4-nonanoylbenzenesulfonic acid and 4-(3,5,5-trimethylhexanoyl)benzenesulfonic acid. The use of acids of the general formula III wherein X represents sulfur is preferred.

The quantity of the acid with a $pKa < 2$ present in the reaction mixture is preferably 0.01—150, more particularly 0.1—100, and most preferably 1—50 equivalents per gram atom of palladium. The acid can optionally be formed in situ, for example by hydrolysis of an ester, for instance such as an alkyl ester of a sulfonic acid, or by reacting a ketone with $SO_2$ and water.

The carbonylation of the olefinically unsaturated compounds should be carried out in the presence both of the phosphine mentioned and the acid as herienbefore defined, preferably in a ratio of at least 2 mol phosphine per equivalent acid with $pKa < 2$.

In the carbonylation according to the invention the presence of small quantities (0.01 to 0.4 mol per mol of olefin) of carboxylic acids in the reaction mixture can be tolerated.

The process of the invention is carried out using an aromatic hydrocarbon as reaction medium. Examples of suitable aromatic hydrocarbons are benzene, toluene, o-, m- or p-xylene, ethylbenzene, pentylbenzene, mesitylene, chlorobenzene, cumene, anisole and diphenyl ethers.

In the process according to the invention the carbon monoxide may be used pure or diluted with an inert gas, such as nitrogen, noble gases or carbon dioxide. The presence of hydrogen in amounts of up to 20 %v is not detrimental to the carbonylation reaction. Preference is given to the use of carbon monoxide or a carbon monoxide containing gas which contains 0.05 to 20 %v of hydrogen.

The process according to the invention is preferably carried out at a temperature in the range between 50 and 200°C, in particular between 80 and 160°C. The overall pressure preferably lies between 50 and 75 bar gauge.

The molar ratio of the olefinically unsaturated compound to the hydroxy sulfonate may lie between 0.1:1 and 10:1, excess of olefinic double bonds, with the molar ratio lying between 1.1:1 and 5:1, being preferred.

The process according to the invention may be carried out batchwise, continuously or semi-continuously. Because of the easy separation of solid reaction products from the liquid reaction medium comprising the homogeneous catalytic system the process is particularly suitable for continuous operation.

Example I

A 250 ml magnetically stirred Hastelloy C autoclave (Hastelloy is a trade mark) was charged with 100 ml toluene, 250 mmol octene-1, 75 mmol sodium 4-hydroxybenzene sulfonate, 0.2 mmol palladium acetate, 40 mmol triphenylphosphine, 8 mmol sulfuric acid and 22 mmol nonanoic acid. The used sodium 4-hydroxybenzene sulfonate had been dried by azeotropic distillation with toluene. The autoclave was flushed with carbon monoxide, filled with carbon monoxide at a pressure of 50 bar, sealed and heated to a temperature of 140°C. After a reaction time of 16 hours the slurry obtained was filtrated. The filtrated solid product was washed with toluene and dried. The dried product was analyzed by $C_{13}$—NMR (in aqueous solution) yielding the composition of the product in %mol. The solid product appeared to consist exclusively of the produced carboxylic acid esters and unreacted sodium 4-hydroxybenzene sulfonate. The total amount of carboxylic acid esters in %mol gives the conversion of sodium 4-hydroxybenzene sulfonate to the esters in %mol.

In addition the reaction was carried out analogously in further Experiments 2—6. The data and results of the Experiments 1—6 are given in Table I. In Experiment 5 0.8 mmol Pd-acetate were employed in place of 0.2 mmol. In Experiment 6 1.3 mmol Pd-acetate, 150 mmol sodium 4-hydroxybenzene sulfonate and 150 ml cumene were employed in place of 0.8 mmol Pd-acetate, 75 mmol sodium 4-hydroxybenzene sulfonate and 100 ml toluene.

TABLE I

| Exp. No. | Temp., °C | Reaction time, h | Olefin (mmol) | Promotor (mmol) | Carboxylic acid added (mmol) | Conversion of Na-4-hydroxy-benzene sulfonate to esters, %mol |
|---|---|---|---|---|---|---|
| 1 | 140 | 16 | octene-1 (250) | $H_2SO_4$ (8) | nonanoic acid (22) | 92 |
| 2 | 135 | 5 | octene-1 (250) | p-toluene sulfonic acid (8) | none | 52 |
| 3 | 135 | 5 | octene-1 (250) | p-toluene sulfonic acid (8) | nonanoic acid (22) | 88 |
| 4 | 150 | 8 | 2,4,4-tri-methylpentene-1 (250) | p-toluene sulfonic acid (8) | nonanoic acid (22) | 52 |
| 5 | 135 | 16 | 2,4,4-tri-methylpentene-1 (250) | p-toluene sulfonic acid (8) | 3,5,5-tri-methyl hexa-noic acid (22) | 64 |
| 6 | 120 | 5.5 | octene-1 (270) | p-tolune sulfonic acid (8) | none | 79 |

Example II

In this example a series of 10 batch reactions is described with recycle of the catalytic system.

For the first batch a 300 ml magnetically stirred Hastelloy C autoclave was charged with 150 ml cumene as solvent, 75 mmol sodium 4-hydroxybenzene sulfonate and 130 mmol octene-1 as reactants, 0.44 mmol palladium acetate and 40 mmol triphenylphosphine as catalyst and 8.4 mmol 4-nonanoylbenzenesulfonic acid as promoter and 22 mmol nonanoic acid. The sodium 4-hydroxybenzene sulfonate had been dried by azeotropic distillation with toluene. The autoclave was flushed and filled with carbon monoxide at a pressure of 50 bar, sealed and heated to a temperature of 120°C. After a reaction time of 3 hours and cooling, the resultant slurry was filtrated and the separated solid product was washed with methylethyl ketone, recovered and dried. The composition of the solid product in %mol was obtained by $C_{13}$—NMR analysis (in aqueous solution), which showed that the produced carboxylic acid esters and sodium 4-hydroxybenzene sulfonate were the only components of the solid product. The conversion of sodium 4-hydroxybenzene sulfonate to the ester products is given by the total amount of ester products formed in %mol.

The filtrate containing cumene solvent, catalyst and methylethyl ketone washing liquid was subjected to distillation to remove unreacted olefin and the washing liquid. The resultant distillation bottom fraction was employed as catalyst solution for the second batch.

The autoclave was charged with the recycle catalyst solution and fresh reactants (75 mmol sodium 4-hydroxybenzene sulfonate and 130 mmol octene-1). The reacton procedure, the recovery of the solid product and of the recycle catalyst solution were carried out in this second batch as described for the first batch.

Subsequently, batches 3—10 were carried out in an identical manner, with the single exception that in batches 8 and 10 fresh amounts of 2 g, respectively 1 g of 4-nonanoylbenzene sulfonic acid promotor were incorporated into the catalyst solution. The data and results of the series batch reactions 1—10 are given in Table II.

TABLE II

| Batch No. | 1 | 2 | 3 | 4 | 5 | 6 | 7 | 8 | 9 | 10 |
|---|---|---|---|---|---|---|---|---|---|---|
| Reaction time, h | 3 | 5 | 4.5 | 5 | 2 | 6.5 | 8 | 4.5 | 7.5 | 5.5 |
| Conversion of Na-4-hydroxybenzene-sulfonate to esters, %mol | 86 | 79 | 91 | 92 | 76 | 88 | 80 | 97 | 96 | 77 |

Example III

In this example a series of 9 batch reactions is described with recycle of the catalytic system.

For the first batch a 300 ml magnetically stirred Hastelloy C autoclave was charged with 150 ml cumene as solvent, 135 mmol sodium 4-hydroxybenzene sulfonate and 260 mmol octene-1 as reactants, 1.4 mmol palladium acetate and 40 mmol triphenylphosphine as catalyst an 9 mmol p-toluene sulfonic acid. The autoclave was flushed and filled with carbon monoxide at a pressure of 63 bar, sealed and heated to a temperature of 120°C. After a reaction time of 4 hours and cooling, the resultant slurry was filtrated and the separated solid product was washed with methylethyl ketone, recovered and dried. The composition of the solid product in %mol was obtained by $C_{13}$—NMR analysis (in aqueous solution), which showed that the produced carboxylic acid esters and sodium 4-hydroxybenzene sulfonate were the only components of the solid product. The conversion of sodium 4-hydroxybenzene sulfonate to the ester products is given by the total amount of ester products formed in %mol.

The filtrate containing cumene solvent, catalyst and methylethyl ketone washing liquid was subjected to distillation to remove unreacted olefin and the washing liquid. The resultant distillation bottom fraction was employed as catalyst solution for the second batch.

The autoclave was charged with the recycle catalyst solution, fresh reactants (262 mmol octene-1 and 152 mmol sodium 4-hydroxybenzene sulfonate) and an additional amount of the promotor (2.0 mmol p-toluene sulfonic acid). The autoclave was flushed with carbon monoxide and filled with a mixture of carbon monoxide and hydrogen at a pressure of 63 bar (partial pressure of carbon monoxide 58 bar and partial pressure of hydrogen 5 bar), sealed and heated to a temperature of 120°C. After a reaction time of 6.5 hours the solid product and the recycle catalyst solution were recovered as described for the first batch.

Subsequently batches 3—9 were carried out in a manner identical to batch 2. The data and results of the batch reactions 1—9 are given in Table III.

TABLE III

| Batch No. | 1 | 2 | 3 | 4 | 5 | 6 | 7 | 8 | 9 |
|---|---|---|---|---|---|---|---|---|---|
| Octene-1, mmol | 260 | 262 | 260 | 260 | 260 | 260 | 263 | 264 | 195 |
| Sodium 4-hydroxybenzene sulfonate, mmol | 153 | 152 | 155 | 155 | 153 | 155 | 152 | 153 | 114 |
| p-Toluene sulfonic acid, mmol | 1.6 | 2.0 | 2.0 | 2.0 | 2.0 | 2.0 | 2.0 | 2.0 | 1.0 |
| CO: partial pressure, bar | 63 | 58 | 55 | 53 | 55 | 53 | 56 | 55 | 55 |
| $H_2$: partial pressure, bar | = | 5 | 5 | 10 | 10 | 10 | 10 | 10 | 10 |
| Reaction time, h | 4 | 6.5 | 6 | 5.5 | 3.5 | 5 | 3 | 6 | 6 |
| Conversion of Na-4-hydroxybenzene sulfonate to esters, % mol | 88 | 90 | 91 | 83 | 93 | 97 | 91 | 100 | 97 |

**Claims**

1. A process for the preparation of carboxylic acid esters of hydroxy sulfonates, characterized in that an olefinically unsaturated compound is carbonylated with carbon monoxide in the presence of an aromatic hydrocarbon reaction medium, a hydroxy sulfonate of the general formula (I) HO—R—$SO_3M$, wherein R represents a divalent optionally substituted hydrocarbon group and M represents a metal of Group I and II of the Periodic Table of the Elements, a palladium catalyst, at least 5 mol of a phosphine of the general formula (II) $PR^1R^2R^3$, wherein $R^1$, $R^2$ and $R^3$ each represent an optionally substituted aryl group, per gram atom of palladium and as promotor an acid having a pKa $< 2$ except hydrohalogenic and carboxylic acids, and in that the obtained solid carboxylic acid ester of hydroxy sulfonate is separated from the reaction mixture.

2. A process as claimed in claim 1, chaacterized in that an acid with a non-coordinating anion is used as promotor.

3. A process as claimed in claim 1 or 2, characterized in that an acid of the general formula (III)

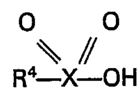

wherein X represents sulfur or chlorine and, if X is chlorine, $R^4$ represents oxygen and, if X is sulfur, $R^4$ represents an OH group or an optionally substituted hydrocarbon group, is used as promotor.

4. A process as claimed in claim 3, characterized in that X is sulfur.

5. A process as claimed in any one of claims 1 to 4, characterized in that the acid is present in a quantity of 0.01—150 equivalents per gram of palladium.

6. A process as claimed in any one of claims 1 to 5, characterized in that the aryl groups $R^1$, $R^2$ and $R^3$ are phenyl groups.

7. A process as claimed in any one of claims 1 to 6, characterized in that at least 10 mol phosphine is used per gram atom palladium.

8. A process as claimed in any one of claims 1 to 7, characterized in that at least 2 mol phosphine is used per equivalent acid with pKa $< 2$.

9. A process as claimed in any one of claims 1 to 8, characterized in that the olefinically unsaturated compound is a substituted or unsubstituted alkene or cycloalkene having 2—30 carbon atoms and 1—3 double bonds.

10. A process as claimed in any one of claims 1—9, characterized in that the divalent hydrocarbon group R is a phenylene group.

**Patentansprüche**

1. Verfahren zur Herstellung von Carbonsäureestern von Hydroxysulfonaten, dadurch gekennzeichnet, daß eine olefinisch ungesättigte Verbindung mit Kohlenmonoxid carbonyliert wird in Gegenwart eines aromatischen Kohlenwasserstoff-Reaktionsmediums, eines Hydroxysulfonats der allgemeinen Formel (I) HO—R—$SO_3M$, wobei R eine zweiwertige, gegebenenfalls substituierte, Kohlenwasserstoffgruppe und M ein Metall der Gruppe I oder II des Periodensystems bedeutet, eines Palladiumkatalysators, mindestens 5 mol eines Phosphins der allgemeinen Formel (II) $PR^1R^2R^3$, wobei $R^1$, $R^2$ und $R^3$ jeweils eine, gegebenenfalls substituierte, Arylgruppe bedeuten, pro g-Atom Palladium und als Promotor einer Säure mit einem pKa-Wert $< 2$, mit Ausnahme von Halogenwasserstoff- und Carbonsäuren, und daß der erhaltene feste Carbonsäureester von Hydroxysulfonat von dem Reaktionsgemisch abgetrennt wird.

2. Verfahren nach Anspruch 1, dadurch gekennzeichnet, daß eine Säure mit einem nichtkoordinierenden Anion als Promotor verwendet wird.

3. Verfahren nach Anspruch 1 oder 2, dadurch gekennzeichnet, daß eine Säure der allgemeinen Formel (III)

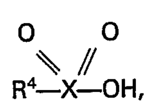

in der X Schwefel oder Chlor bedeutet und, wenn X Chlor ist, $R^4$ Sauerstoff bedeutet und, wenn X Schwefel ist, $R^4$ eine OH-Gruppe oder eine gegebenenfalls substituierte Kohlenwasserstoffgruppe bedeutet, als Promotor verwendet wird.

4. Verfahren nach Anspruch 3, dadurch gekennzeichnet, daß X Schwefel ist.

5. Verfahren nach einem der Ansprüche 1 bis 4, dadurch gekennzeichnet, daß die Säure in einer Menge von 0,01 bis 150 Äquivalent pro g-Atom Palladium vorhanden ist.

6. Verfahren nach einem der Ansprüche 1 bis 5, dadurch gekennzeichnet, daß die Arylgruppen $R^1$, $R^2$ und $R^3$ Phenylgruppen sind.

7. Verfahren nach einem der Ansprüche 1 bis 6, dadurch gekennzeichnet, daß mindestens 10 mol Phosphin pro g-Atom Palladium verwendet werden.

8. Verfahren nach einem der Ansprüche 1 bis 7, dadurch gekennzeichnet, daß mindestens 2 mol Phosphin pro Äquivalent Säure mit pKa $< 2$ verwendet werden.

9. Verfahren nach einem der Ansprüche 1 bis 8, dadurch gekennzeichnet, daß die olefinisch ungesättigte Verbindung ein substituiertes oder unsubstituiertes Alken oder Cycloalken mit 2 bis 30 Kohlenstoffatomen und 1 bis 3 Doppelbindungen ist.

10. Verfahren nach der Ansprüche 1 bis 9, dadurch gekennzeichnet, daß der zweiwertige Kohlenwasserstoffrest R eine Phenylengruppe ist.

**Revendications**

1. Un procédé de préparation d'esters d'acides carboxyliques d'hydroxysulfonates, caractérisé en ce qu'un composé oléfiniquement insaturé est carbonylé avec de l'oxyde de carbone en présence d'un milieu réactionnel hydrocarbure aromatique, d'un hydroxy sulfonate de la formule générale (I) HO—R—$SO_3M$, où R représente un groupe d'hydrocarbure divalent éventuellement substitué et M représente un métal des groupes I et II du tableau périodique des éléments, d'un catalyseur au palladium, d'au moins 5 moles d'une phosphine de la formule générale (II) $PR^1R^2R^3$, où $R^1$, $R^2$ et $R^3$ représentent chacun un groupe aryle éventuellement substitué, par atome-gramme de palladium et, comme promoteur, d'un acide ayant un pKa inférieur à 2, à l'exception des acides halogénhydriques et carboxyliques, et que l'ester d'acide carboxylique d'hydroxysulfonate solide obtenu est séparé du mélange de réaction.

2. Un procédé selon la revendication 1, caractérisé en ce qu'un acide ayant un anion non-coordinateur est utilisé comme promoteur.

3. Un procédé selon la revendication 1 ou 2, caractérisé en ce q'un acide de la formule générale (III)

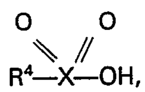

où X représente du soufre ou du chlore et, si X est du chlore, $R^4$ représente de l'oxygène et, si X est du soufre, $R^4$ représente un groupe OH ou un groupe d'hydrocarbure éventuellement substitué, est utilisé comme promoteur.

4. Un procédé selon la revendication 3, caractérisé en ce que X est du soufre.

5. Un procédé selon l'une quelconque des revendications 1 à 4, caractérisé en ce que l'acide est présent à raison de 0,01—150 équivalents par atome-gramme de palladium.

6. Un procédé selon l'une quelconque des revendications 1 à 5, caractérisé en ce que les groupes aryle $R^1$, $R^2$ et $R^3$ sont des groupes phényle.

7. Un procédé selon l'une quelconque des revendications 1 à 6, caractérisé en ce qu'on utilise au moins 10 moles de phosphine par atome-gramme de palladium.

8. Un procédé selon l'une quelconque des revendications 1 à 7, caractérisé en ce qu'on utilise au moins 2 moles de phosphine par équivalent d'acide ayant un pKa inférieur à 2 .

9. Un procédé selon l'une quelconque des revendications 1 à 8, caractérisé en ce que le composé oléfiniquement insaturé est un alcène ou cycloalcène substitué ou non ayant 2—30 atomes de carbone et 1—3 doubles liaisons.

10. Un procédé selon l'une quelconque des revendications 1—9, caractérisé en ce que le groupe d'hydrocarbure divalent R est un groupe phénylène.